# EUROPEAN PATENT APPLICATION

(11) **EP 4 349 278 A1**
(43) Date of publication of application: **10.04.2024**
(21) Application number: 21942709.3
(22) Date of filing: 02.11.2021
(51) Int. Cl.: A61B 17/12

(54) **INTRATUMOR TURBULENT FLOW DEVICE**

(30) Priority: 24.05.2021 CN 202110566313
(71) Applicant: Beijing Taijieweiye Technology Co., Ltd., Beijing 101204 (CN)
(72) Inventor: MU, Lei, Beijing 101204 (CN); TANG, Hang, Beijing 101204 (CN); XU, Yongsong, Beijing 101204 (CN); CHENG, Wenjie, Beijing 101204 (CN); CHEN, Fei, Beijing 101204 (CN); FAN, Wenji, Beijing 101204 (CN); ZHANG, Hongqing, Beijing 101204 (CN); WU, Yongzhao, Beijing 101204 (CN)
(74) Representative: Glück Kritzenberger Patentanwälte PartGmbB
(86) International application number: PCT/CN2021/128042
(87) International publication number: WO 2022/247145

(57) **Abstract**

The present invention relates to an intratumor turbulent flow device, comprising: a turbulent flow net disc of a double-layer metal wire braided structure, wherein a developing mark is provided at the bottom in the center of the turbulent flow net disc, and metal wires in the center of the turbulent flow net disc are fixed in the developing mark; the center of the turbulent flow net disc is protruded upwards; the bottom end of the developing mark does not exceed the lowest end of the turbulent flow net disc; a release portion, a distal end of the release portion being fixedly connected to the developing mark; a pushing guide wire, the pushing guide wire being connected to the turbulent flow net disc by means of the release portion, and being used for pushing the turbulent flow net disc; and a microcatheter used for transporting the turbulent flow net disc. The pushing guide wire pushes, into a tumor cavity of the aneurysm along the microcatheter, the turbulent flow net disc compressed to be bundle-shaped. The turbulent flow net disc expands in the tumor cavity and is fitted to the inner wall of the tumor cavity. The release portion is broken by electrolysis or electric heating, so that the center of the turbulent flow net disc is restored to be protruded for pulling the developing mark into the tumor cavity, and thus the developing mark is prevented from forming an embolism in the blood vessel.

## Description

### CROSS-REFERENCE TO RELATED APPLICATION

This application claims priority to Chinese Patent Application No. 202110566313.3 filed to China Patent Office on May 24, 2021 and entitled "INTRATUMOR TURBULENT FLOW DEVICE".

### BACKGROUND OF THE INVENTION

### 1. Technical Field

The present invention relates to the field of medical devices, and in particular to an intratumor turbulent flow device.

### 2. Description of Related Art

Aneurysm refers to the persistent dilation of a localized arterial segment caused by a lesion or an injury occurring to the side wall of an artery. In Chinese population, the detection rate of intracranial aneurysm reaches about 9%, and the incidence of intracranial aneurysm in the population with the intracranial aneurysm is approximately 3.2%. The intracranial aneurysms can generally be classified into two major classes, including narrow-neck aneurysms (with a neck less than 4 mm) and wide-neck aneurysms (with a neck greater than or equal to 4 mm or a dome-to-neck ratio less than 2), according to the size of the neck. At present, therapies for intracranial aneurysms include an interventional therapy, surgical clipping, conservative treatment, or the like. In recent years, with the continuous development of interventional radiology and the emergence of new materials and technologies, the interventional therapy has taken an irreplaceable position in the treatment of intracranial aneurysms, showing a tendency to massively replace the surgical procedures.

Coils emerge to represent an important milestone in the development of interventional therapies for intracranial aneurysms, but the coil embolization technology has the following inherent defects: first, higher operation requirements are put forward to the surgeons, because the effect of the coil in the treatment of intracranial aneurysms is associated with its level of dense embolization, and the coils need to be placed as many as possible during the operation to strive for dense embolization; second, the recurrence rate of aneurysms is higher, since more than 1/3 of patients has undergone recanalization in a medium-to long-term clinical follow-up, and even for aneurysm patients with complete embolization, more than 26.4% of the patents have aneurysm recanalization; and third, the coil embolization therapy for the aneurysms poses a higher risk of delayed rupturing and bleeding of the aneurysms.

In order to overcome these defects of the coil therapy for intracranial aneurysms, the concept of parent artery reconstruction was proposed, and a blood flow diversion device emerged at the right moment. The blood flow diversion device is a stent with low mesh porosity. This dense-mesh stent is disposed inside a parental artery, rather than inside a cavity of an aneurysm, to haemodynamically reconstruct the parental artery to induce spontaneous thrombosis inside the aneurysm and reshape the inner surface of a vascular lumen by means of a neovascular endomembrane on the surface of the neck of the aneurysm, thereby finally achieving the purpose of curing the aneurysm. Since the first blood flow diversion device Pipeline was reported in 2007, the blood flow diversion device represented by Pipeline has undergone more than ten years of updates and iterations, whereby the usability and therapeutic effect of the blood flow diversion device have been improved, and moreover, after long-term clinical verification, the indications of the blood flow diversion have been expanded from initial large or huge aneurysms to small-to medium-sized wide-neck aneurysms. The clinical use of the blood flow diversion has been generally recognized by physicians. However, during the use of the blood flow diversion device, there are still some insurmountable defects as follows: first, a patient with the implanted blood flow diversion device needs to take antiplatelet drugs for a long term, resulting in the latent risk of intracranial bleeding; second, occlusion is caused by the coverage of branch blood vessels, and in particular, complications such as narrow vascular occlusion easily occur during the treatment of a wide-neck aneurysm at a bifurcated site; third, intracavity thrombosis induced by the blood flow diversion device is one of the most serious complications during the use of the blood flow diversion device, and requires a double-antibody therapy before and after the surgery; and fourth, the blood flow diversion device has a poor opening effect in a complex blood vessel, leading to vascular stenosis or even occlusion.

In addition, it is still very difficult to administrate an endovascular interventional therapy to a wide-neck aneurysm at a bifurcated site on an intracranial artery, and such an aneurysm accounts for about 2.9%-7.1% of all the intracranial aneurysms. For the endovascular therapy against the wide-neck aneurysm at the bifurcated site on the blood vessel and other wide-neck aneurysms, the biggest problems lie in the stable filling of a coil, stent-assisted coiling, impacts of the blood flow diversion device to branch blood vessels, and aneurysm recurrence.

A special class of blood flow diversion devices, represented by Woven EndoBridge (WEB), has been successively developed. Such devices are implanted into the cavities of aneurysms to perform blood flow reconstruction, without affecting the parental artery. Such devices have the following main features: first, the advantage of requiring no antiplatelet therapy after surgery; and second, better applicability to wide-neck aneurysms, in particular the aneurysm at the bifurcated site. Such devices are an important supplement to the blood flow diversion devices in the aneurysm treatment. However, such devices are spherical in overall form to lead to high difficulty in filling in the aneurysms, and still have problems such as the mass effect like that of the coils, and residues at the site of the neck of an aneurysm. When such devices are disposed in the aneurysms, their proximal marks bulge significantly, and may easily cause thrombotic events after entering a vascular lumen.

### BRIEF SUMMARY OF THE INVENTION

In order to overcome the defects of the prior art, an object of the present invention is to provide an intratumor turbulent flow device, in which a mesh disc structure braided from developable double-layer metal wires is used, the metal wires can slide relative to each other without a fixed point, and a center of the mesh disc is fixed within a developing mark. The turbulent flow mesh disc, which is compressed into a bundle shape, may be pushed into a cavity of an aneurysm along a microcatheter by means of a guide wire; a release portion connecting the guide wire and the turbulent flow mesh disc is broken, leaving the turbulent flow mesh disc in the cavity of the aneurysm; the turbulent flow mesh disc expands in the cavity of the aneurysm and is closely fitted to the inner wall of the cavity of the aneurysm, blocking a blood flow from entering or exiting the cavity of the aneurysm to induce an embolism inside the cavity of the aneurysm; and meanwhile, the center of the turbulent flow mesh disc is restored to a bulge to pull a proximal end of the developing mark into a neck of the cavity, thereby preventing intravascular embolism.

To achieve the object above, the present invention provides an intratumor turbulent flow device, including:
a turbulent flow mesh disc, wherein the turbulent flow mesh disc is of a double-layer metal wire braided structure, a developing mark is provided at a bottom in a center of the turbulent flow mesh disc, metal wires in the center of the turbulent flow mesh disc are fixed within the developing mark, the center of the turbulent flow mesh disc bulges upwards, and a bottom end of the developing mark does not exceed a lowest end of the turbulent flow mesh disc;
a release portion, wherein a distal end of the release portion is fixedly connected to the developing mark;
a pushing guide wire, wherein the pushing guide wire is connected to the turbulent flow mesh disc by means of the release portion, and is configured to push the turbulent flow mesh disc; and
a microcatheter for delivering the turbulent flow mesh disc, wherein
the pushing guide wire pushes the turbulent flow mesh disc, which is compressed into a bundle shape, into a cavity of an aneurysm along the microcatheter, the turbulent flow mesh disc expands in the cavity and is closely fitted to an inner wall of the cavity, and the release portion is broken by electrolysis or electric heating, such that the center of the turbulent flow mesh disc is restored to a bulge for pulling the developing mark into the cavity.

Preferably, the metal wires have a number of 36, 48, 64, 72, 96, 128, or 144; and
the plurality of metal wires can slide relative to each other without a fixed point.

Preferably, the metal wires are made of a material of nitinol, platinum-core nickel-titanium, or 35NLT alloy; and
the metal wires may be developable metal wires.

Preferably, the developing mark is a solid ring made of gold, tantalum, platinum-iridium alloy, or platinum-tungsten alloy.

Preferably, an outer edge of the turbulent flow mesh disc has a height not greater than a height of the bulge in the center of the turbulent flow mesh disc.

Preferably, the turbulent flow mesh disc has a height ranging from 5% to 40% of a width of the turbulent flow mesh disc.

An embodiment of the present invention provides an intratumor turbulent flow device, in which a mesh disc structure braided from developable double-layer metal wires is used, the metal wires can slide relative to each other without a fixed point, and a center of the mesh disc is fixed within a developing mark. The turbulent flow mesh disc, which is compressed into a bundle shape, may be pushed into a cavity of an aneurysm along the microcatheter by means of a guide wire; a release portion connecting the guide wire and the turbulent flow mesh disc is broken, leaving the turbulent flow mesh disc in the cavity of the aneurysm; the turbulent flow mesh disc expands in the cavity of the aneurysm and is closely fitted to the inner wall of the cavity of the aneurysm, blocking the blood flow from entering or exiting the cavity of the aneurysm to induce an embolism inside the cavity of the aneurysm; and meanwhile, the center of the turbulent flow mesh disc is restored to a bulge to pull a proximal end of the developing mark into a neck of the cavity of the aneurysm, thereby preventing intravascular embolism.

### BRIEF DESCRIPTION OF THE SEVERAL VIEWS OF THE DRAWINGS

FIG. 1 is a schematic structural diagram of an intratumor turbulent flow device with a turbulent flow mesh disc expanded, according to an embodiment of the present invention;
FIG. 2 is a schematic diagram of an intratumor turbulent flow device delivered in a microcatheter, according to an embodiment of the present invention;
FIG. 3 is a schematic diagram of an intratumor turbulent flow device released from a microcatheter, according to an embodiment of the present invention;
FIG. 4 is a schematic diagram of an intratumor turbulent flow device completely released and separated from a microcatheter, according to an embodiment of the present invention; and
FIG. 5 is a schematic diagram of an intratumor turbulent flow device applied in an aneurysm, according to an embodiment of the present invention.

### DETAILED DESCRIPTION OF THE INVENTION

The technical solutions of the present invention will be further described in details below in combination with the accompanying drawings and embodiments.

An embodiment of the present invention provides an intratumor turbulent flow device, in which a mesh disc structure braided from developable double-layer metal wires is used, the metal wires can slide relative to each other without a fixed point, and a center of the mesh disc is fixed within a developing mark. The turbulent flow mesh disc, which is compressed into a bundle shape, may be pushed into a cavity of an aneurysm along the microcatheter by means of a guide wire; a release portion connecting the guide wire and the turbulent flow mesh disc is broken, leaving the turbulent flow mesh disc in the cavity of the aneurysm; the turbulent flow mesh disc expands in the cavity of the aneurysm and is closely fitted to the inner wall of the cavity of the aneurysm, blocking the blood flow from entering or exiting the cavity of the aneurysm to induce an embolism inside the cavity of the aneurysm; and meanwhile, the center of the turbulent flow mesh disc is restored to a bulge to pull a proximal end of the developing mark into a neck of the cavity of the aneurysm, thereby preventing intravascular embolism.

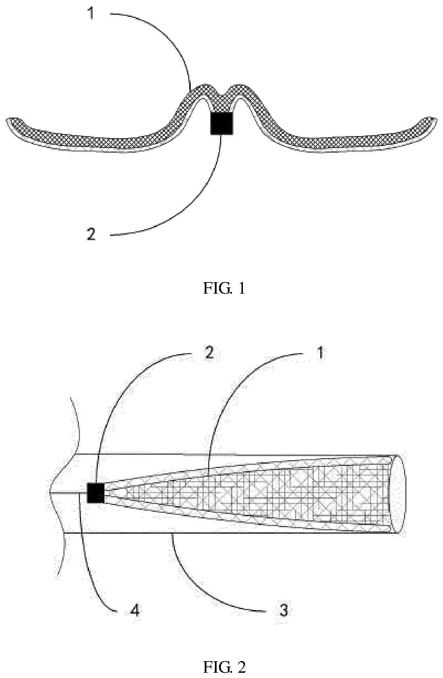

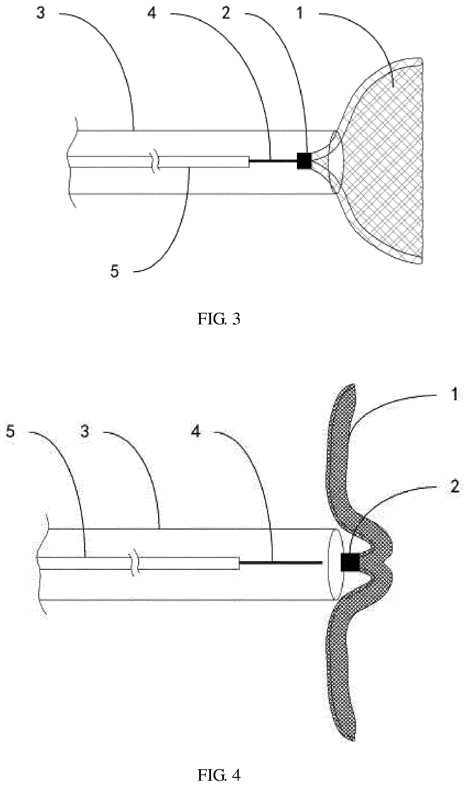

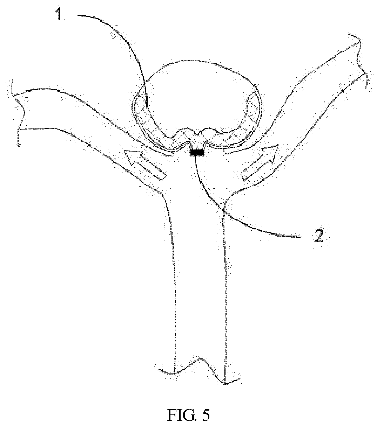

FIG. 1 is a schematic structural diagram of an intratumor turbulent flow device with a turbulent flow mesh disc expanded, according to an embodiment of the present invention. FIG. 2 is a schematic diagram of an intratumor turbulent flow device delivered in a microcatheter, according to an embodiment of the present invention. FIG. 3 is a schematic diagram of an intratumor turbulent flow device released from a microcatheter, according to an embodiment of the present invention. FIG. 4 is a schematic diagram of an intratumor turbulent flow device completely released and separated from a microcatheter, according to an embodiment of the present invention. FIG. 5 is a schematic diagram of an intratumor turbulent flow device applied in an aneurysm, according to an embodiment of the present invention. As shown in FIGs. 1-5, the intratumor turbulent flow device includes a turbulent flow mesh disc 1, a developing mark 2, a microcatheter 3, a release portion 4, and a pushing guide wire 5.

The turbulent flow mesh disc 1 is a mesh-disc-shaped device of a double-layer structure braided from metal wires, and the metal wires in the center part of the turbulent flow mesh disc 1 are fixed within a developing mark 2 below a center point of the turbulent flow mesh disc 1. The center of the turbulent flow mesh disc 1 is made into an upward bulging shape, and thus may hide the developing mark 2 inside the bulging part. That is, the bottom end of the developing mark 2 does not exceed the lowest end of the turbulent flow mesh disc 1. When the turbulent flow mesh disc 1 is expanded, the outer edge of the turbulent flow mesh disc 1 has a height not greater than that of the center bulge of the turbulent flow mesh disc 1, and the height of the turbulent flow mesh disc 1 ranges from 5% to 40% of the width of the turbulent flow mesh disc 1.

The metal wires for making the turbulent flow mesh disc 1 are made of nitinol, 10%-30% platinum-core nickel-titanium, or 35NLT alloy, and the number of the metal wires may be 36, 48, 64, 72, 96, 128, or 144. During the braiding of the turbulent flow mesh disc 1 from the metal wires, the metal wires may slide relative to each other without a fixed point, and the center of the braided the turbulent flow mesh disc 1 is bound into the developing mark 2. The metal wires may be developable metal wires capable of developing during an operation.

The turbulent flow mesh disc 1 may be compressed into a bundle shape to facilitate pushing, and the shape of the expanded low disruption mesh disc may comply with the inner wall of a cavity of an aneurysm to block a neck of an aneurysm and prevent blood from entering or exiting the aneurysm, thereby implementing the embolization therapy for the aneurysm.

The developing mark 2 is a solid ring made of gold, tantalum, platinum-iridium alloy, or platinum-tungsten alloy, and thus is an X-ray proof mark. The developing mark 2 is a mark configured to indicate a release position for the intratumor turbulent flow device during an operation, and is simultaneously a mark point of a position for recovering the intratumor turbulent flow device.

The microcatheter 3 is configured to build a delivery passage for the turbulent flow mesh disc 1 during an operation.

The pushing guide wire 5 is configured to push the turbulent flow mesh disc 1 inside the delivery passage built by the microcatheter 3; the pushing guide wire 5 is connected to the turbulent flow mesh disc 1 by means of the release portion 4; and the turbulent flow mesh disc 1 may be compressed into a bundle shape and disposed inside the microcatheter 3, and pushed by the pushing guide wire 5 into the cavity of the aneurysm. Here, the release portion 4 may be broken under the action of an electrolytic separation system or an electric heating separation system, to deliver the turbulent flow mesh disc 1 into the cavity of the aneurysm.

In the intratumor turbulent flow device provided in an embodiment of the present invention, a turbulent flow mesh disc with an outer diameter of 5 mm is made by the steps of: braiding a braided tube with an outer diameter of 5 mm from 48 pieces of 10% platinum-core nickel-titanium of 0.0015 in by a braiding machine, and carrying out preliminary heat shaping to obtain a braided tube of 5 mm; and folding the braided tube inwards to form a double-layer braided structure, and shaping the double-layer braided structure to form a "hat"-like bulge by a heat shaping tooling, to obtain the turbulent flow mesh disc with the center bulging like a "hat". The double-layer braided metal wires of the turbulent flow mesh disc are bound together by the developing mark, and fixed by means of glue curing or riveting, and the turbulent flow mesh disc is connected to the release portion by means of the developing mark.

In the embodiment described above, the braided tube with the outer diameter of 5 mm may also be: a braided tube with an outer diameter of 9 mm braided from 64 pieces of 15% platinum-core nickel-titanium of 0.0015 in, a braided tube with an outer diameter of 15 mm braided from 72 pieces of 20% platinum-core nickel-titanium of 0.001 in, or a braided tube with an outer diameter of 7 mm braided from 48 pieces of nickel-titanium alloy wires of 0.001 inch and 12 pieces of tantalum wires of 0.0015 in, or the like.

The intratumor turbulent flow device provided in an embodiment of the present invention concretely works as follows:

an access to the aneurysm is first built by means of the microcatheter; the turbulent flow mesh disc compressed into a bundle shape is disposed inside the microcatheter, and is pushed into the cavity of the aneurysm by means of the pushing guide wire; the turbulent flow mesh disc is released into the cavity of the aneurysm by means of the microcatheter and is expanded to closely fit the inner wall of the cavity of the aneurysm; the release portion is broken under the action of the electrolytic separation system or the electric heating separation system; the center of the turbulent flow mesh disc is restored to a bulging state to pull the developing mark into and hide the same in the neck of the aneurysm, thereby preventing the developing mark from inducing embolism in a vascular lumen.

Compared with the prior art, the embodiment of the present invention provides an intratumor turbulent flow device, in which a mesh disc structure braided from developable double-layer metal wires is used, the metal wires can slide relative to each other without a fixed point, and a center of the mesh disc is fixed within a developing mark. The turbulent flow mesh disc, which is compressed into a bundle shape, may be pushed into a cavity of an aneurysm along the microcatheter by means of a guide wire; a release portion connecting the guide wire and the turbulent flow mesh disc is broken, leaving the turbulent flow mesh disc in the cavity of the aneurysm; the turbulent flow mesh disc expands in the cavity of the aneurysm and is closely fitted to the inner wall of the cavity of the aneurysm, blocking the blood flow from entering or exiting the cavity of the aneurysm to induce an embolism inside the cavity of the aneurysm; and meanwhile, the center of the turbulent flow mesh disc is restored to a bulge to pull a proximal end of the developing mark into a neck of the cavity of the aneurysm, thereby preventing intravascular embolism. The intratumor turbulent flow device provided by the embodiments of the present invention is soft and flexible, and can comply with the cavity of the aneurysm to be stably disposed inside the cavity of the aneurysm, without affecting the parental artery. Moreover, the turbulent flow device has a very high metal surface coverage rage, and can significantly to change the hemodynamic force in the aneurysm, such that a blood clot can be formed quickly inside the cavity of the aneurysm one the one hand, and on the other hand, endothelialization can be completed more quickly at the neck of the aneurysm, thereby achieving the treatment of the aneurysm. Meanwhile, this device can also minimize the risk of introducing a foreign matter into a blood vessel, which effectively reduces complications, and can reduce the use of anticoagulants after the endothelialization at the neck of the aneurysm.

The objects, technical solutions and advantageous effects of the present invention are further illustrated in detail with the specific embodiments described above. It should be understood that the description above only involves the specific embodiments of the present invention and is not intended to limit the scope of protection of the present invention. Any modifications, equivalent substitutions, improvements or the like made within the spirit and principle of the present invention shall be construed as being included within the protection scope of the present invention.

## Claims

1. An intratumor turbulent flow device, comprising:
a turbulent flow mesh disc, wherein the turbulent flow mesh disc is of a double-layer metal wire braided structure, a developing mark is provided at a bottom in a center of the turbulent flow mesh disc, metal wires in the center of the turbulent flow mesh disc are fixed within the developing mark, the center of the turbulent flow mesh disc bulges upwards, and a bottom end of the developing mark does not exceed a lowest end of the turbulent flow mesh disc;
a release portion, wherein a distal end of the release portion is fixedly connected to the developing mark;
a pushing guide wire, wherein the pushing guide wire is connected to the turbulent flow mesh disc by means of the release portion, and is configured to push the turbulent flow mesh disc; and
a microcatheter for delivering the turbulent flow mesh disc, wherein
the pushing guide wire pushes the turbulent flow mesh disc, which is compressed into a bundle shape, into a cavity of an aneurysm along the microcatheter, the turbulent flow mesh disc expands in the cavity and is closely fitted to an inner wall of the cavity, and the release portion is broken by electrolysis or electric heating, such that the center of the turbulent flow mesh disc is restored to a bulge for pulling the developing mark into the cavity.

2. The intratumor turbulent flow device according to claim 1, wherein the metal wires have a number of 36, 48, 64, 72, 96, 128, or 144; and
the plurality of metal wires can slide relative to each other without a fixed point.

3. The intratumor turbulent flow device according to claim 1, wherein the metal wires are made of a material of nitinol, platinum-core nickel-titanium, or 35NLT alloy; and
the metal wires may be developable metal wires.

4. The intratumor turbulent flow device according to claim 1, wherein the developing mark is a solid ring made of gold, tantalum, platinum-iridium alloy, or platinum-tungsten alloy.

5. The intratumor turbulent flow device according to claim 1, wherein an outer edge of the turbulent flow mesh disc has a height not greater than a height of the bulge in the center of the turbulent flow mesh disc.

6. The intratumor turbulent flow device according to claim 1, wherein the turbulent flow mesh disc has a height ranging from 5% to 40% of a width of the turbulent flow mesh disc.
